# EUROPEAN PATENT APPLICATION

(11) **EP 4 070 804 A1**
(43) Date of publication of application: **12.10.2022**
(21) Application number: 20896317.3
(22) Date of filing: 02.12.2020
(51) Int. Cl.: A61K 35/74, A23L 33/135, A61P 1/02, A61P 31/04

(54) **AGENT FOR PREVENTING OR AMELIORATING PERIODONTAL DISEASE**

(30) Priority: 03.12.2019 JP 2019218905
(71) Applicant: Kao Corporation, Chuo-ku Tokyo 103-8210 (JP)
(72) Inventor: AKATSU, Tomoki, Haga-gun, Tochigi 321-3497 (JP); FUJII, Akihiko, Haga-gun, Tochigi 321-3497 (JP); SHIRAISHI, Akiko, Tokyo 131-8501 (JP); YAMAMOTO, Naoki, Tokyo 131-8501 (JP); SONO, Hatsumi, Haga-gun, Tochigi 321-3497 (JP)
(74) Representative: Vossius & Partner Patentanwälte Rechtsanwälte mbB
(86) International application number: PCT/JP2020/044925
(87) International publication number: WO 2021/112140

(57) **Abstract**

Provided is a material useful for suppressing infection with periodontal bacteria to prevent or ameliorate periodontal diseases. Provided is a periodontal bacterial infection-suppressing agent containing bacterial cells or a bacterial cell culture of Neisseria mucosa as an active component.

## Description

### Field of the Invention

The present invention relates to an agent for preventing or ameliorating periodontal diseases.

### Background of the Invention

Among the more than 600 types of bacteria present in the oral cavity, the cause of periodontal diseases is periodontal bacteria which cause chronic inflammatory diseases. Periodontal bacteria infect the periodontal tissue and invade the cells, to cause gingivitis and further periodontal tissue destruction.

Examples of the existing technique for preventing/ameliorating periodontal diseases include bactericides and antibiotics against periodontal bacteria. However, these techniques can disrupt the normal oral flora and cause the emergence of resistant bacteria due to their strong bactericidal and bacteriostatic effects, which may lead to serious diseases.

In recent years, there have been reported techniques for suppressing the growth of periodontal bacteria by using lactic acid bacteria and bifidobacteria, which are gaining attention as probiotics. For example, there have been reports that specific strains belonging to Lactococcus lactis, Lactobacillus rhamnosus, and Lactobacillus curvatus have the effect of inhibiting oral biofilm formation and the effect of inhibiting the growth of Porphyromonas gingivalis and Fusobacterium nucleatum (Patent Literature 1), lactic acid bacteria belonging to Enterococcus faecium have the effect of inhibiting growth of Porphyromonas gingivalis (Patent Literature 2), and Leuconostoc mesenteroides has the effect of inhibiting biofilm formation by Porphyromonas gingivalis or the like (Patent Literature 3).

Meanwhile, it is known that bacteria of the genus Neisseria such as Neisseria mucosa, Neisseria sicca, Neisseria flava, Neisseria subflava, Neisseria flavescens, and Neisseria elongata are present in the oral cavity as non-pathogenic commensal bacteria, but their functions are not clear.

[Patent Literature 1] JP-A-2010-124772
[Patent Literature 2] JP-A-2006-328052
[Patent Literature 3] JP-A-2010-53062

### Summary of the Invention

The present invention relates to from 1) to 22) below.
1) A periodontal bacterial infection-suppressing agent comprising bacterial cells or a bacterial cell culture of Neisseria mucosa as an active component.
2) An alveolar bone resorption-suppressing agent comprising bacterial cells or a bacterial cell culture of Neisseria mucosa as an active component.
3) An agent for preventing or ameliorating periodontal diseases, comprising bacterial cells or a bacterial cell culture of Neisseria mucosa as an active component.
4) A periodontal bacterial infection-suppressing food, comprising bacterial cells or a bacterial cell culture of Neisseria mucosa as an active component.
5) An alveolar bone resorption-suppressing food, comprising bacterial cells or a bacterial cell culture of Neisseria mucosa as an active component.
6) A food for preventing or ameliorating periodontal diseases, comprising bacterial cells or a bacterial cell culture of Neisseria mucosa as an active component.
7) Use of bacterial cells or a bacterial cell culture of Neisseria mucosa for producing a periodontal bacterial infection-suppressing agent.
8) Use of bacterial cells or a bacterial cell culture of Neisseria mucosa for producing an alveolar bone resorption-suppressing agent.
9) Use of bacterial cells or a bacterial cell culture of Neisseria mucosa for producing an agent for preventing or ameliorating periodontal diseases.
10) Use of bacterial cells or a bacterial cell culture of Neisseria mucosa for producing a periodontal bacterial infection-suppressing food.
11) Use of bacterial cells or a bacterial cell culture of Neisseria mucosa for producing an alveolar bone resorption-suppressing food.
12) Use of bacterial cells or a bacterial cell culture of Neisseria mucosa for producing a food for preventing or ameliorating periodontal diseases.
13) Bacterial cells or a bacterial cell culture of Neisseria mucosa for use in suppressing periodontal bacterial infection.
14) Bacterial cells or a bacterial cell culture of Neisseria mucosa for use in suppressing alveolar bone resorption.
15) Bacterial cells or a bacterial cell culture of Neisseria mucosa for use in preventing or ameliorating periodontal diseases.
16) Non-therapeutic use of bacterial cells or a bacterial cell culture of Neisseria mucosa for suppressing periodontal bacterial infection.
17) Non-therapeutic use of bacterial cells or a bacterial cell culture of Neisseria mucosa for suppressing alveolar bone resorption.
18) Non-therapeutic use of bacterial cells or a bacterial cell culture of Neisseria mucosa for preventing or ameliorating periodontal diseases.
19) A method for suppressing periodontal bacterial infection, comprising: administering bacterial cells or a bacterial cell culture of Neisseria mucosa to a subject in need thereof, or allowing the subject to ingest bacterial cells or a bacterial cell culture of Neisseria mucosa, in an effective amount.
20) A method for suppressing alveolar bone resorption, comprising administering bacterial cells or a bacterial cell culture of Neisseria mucosa to a subject in need thereof, or allowing the subject to ingest bacterial cells or a bacterial cell culture of Neisseria mucosa, in an effective amount.
21) A method for preventing or ameliorating periodontal diseases, comprising administering bacterial cells or a bacterial cell culture of Neisseria mucosa to a subject in need thereof, or allowing the subject to ingest bacterial cells or a bacterial cell culture of Neisseria mucosa, in an effective amount.
22) A method for producing a culture containing EPS of Neisseria mucosa, comprising adding a culture supernatant of Porphyromonas gingivalis to a culture solution of Neisseria mucosa, followed by culturing; and then removing bacterial cells and medium components, followed by collecting.

### Brief Description of the Drawings

[Figure 1] Figure 1 shows the effect of inhibiting infection with periodontal bacteria of Neisseria mucosa, which is dependent on the number of bacterial cells. The MOI (Multiplicity of infection) indicates the number of bacterial cells added.
[Figure 2] Figure 2 shows the effect of inhibiting infection with periodontal bacteria of bacteria of the genus Neisseria.
[Figure 3] Figure 3 shows the effect of inhibiting infection with various periodontal bacteria by Neisseria mucosa.
[Figure 4] Figure 4 shows the effect of increasing the yield of the bacterial cell culture of Neisseria mucosa by adding a culture supernatant of Porphyromonas gingivalis.
[Figure 5] Figure 5 includes photographs of EPS produced by Neisseria mucosa and increased by adding the culture supernatant of Porphyromonas gingivalis.
[Figure 6] Figure 6 shows the effect of inhibiting infection with periodontal bacteria by the bacterial cell culture of Neisseria mucosa.
[Figure 7] Figure 7 shows the effect of suppressing alveolar bone resorption (alveolar bone resorption amount) by Neisseria mucosa.
[Figure 8] Figure 8(A) shows the effect of suppressing alveolar bone resorption by Neisseria mucosa (photographs of the mouse maxillas after test), and Figure 8(B) shows the site where the alveolar bone resorption was measured (arrow: measured site).

### Detailed Description of the Invention

The present invention relates to providing a material useful for suppressing infection with periodontal bacteria to prevent or ameliorate periodontal diseases.

As a result of diligent studies on oral microorganisms, the inventors found that bacterial cells of Neisseria mucosa known as non-pathogenic oral commensal bacteria or a culture thereof suppresses the infection of gingival cells with periodontal bacteria, and exhibits an effect of suppressing alveolar bone resorption on animal models, and is thus useful as a preventive or therapeutic agent for periodontal diseases.

Being a non-pathogenic oral commensal bacterium, the Neisseria mucosa of the present invention can provide an agent for preventing or ameliorating periodontal diseases, which can be ingested not only by healthy people but also elderly people or people after illness over a long period of time.

The Neisseria mucosa used in the present invention is a gram-negative coccus belonging to the genus Neisseria of the family Neisseria and is a non-pathogenic oral commensal bacterium.

Neisseria mucosa can be isolated from human saliva, dental plaque, or the like. Further, it is available from JCM (Neisseria mucosa JCM12992 strain (ATCC19696 strain)).

In the present invention, the "bacterial cells" of Neisseria mucosa may be any of live bacterial cells, wet bacterial cells, dried bacterial cells of Neisseria mucosa. Further, the "bacterial cells" of Neisseria mucosa may be not only live bacterial cells but also dead bacterial cells. Further, the bacterial cells also include bacterial cell components such as cytoplasms and cell wall fractions obtained by treating the bacterial cells with enzymes or physical means.

Examples of the "bacterial cell culture" of Neisseria mucosa include not only a culture containing medium components, bacterial cells, extracellular polymeric substances (EPS), and the like, a culture containing bacterial cells and EPS in which the medium components has been removed from the culture, a culture containing medium components and EPS in which the bacterial cells have been removed from the culture, or a culture containing EPS in which the medium components and the bacterial cells have been removed from the culture.

The bacterial cells or the bacterial cell culture of the present invention can be prepared into any form depending on the purpose of use such as freeze-dried powder, spray-dried powder, and a suspension in a liquid, as needed.

The medium to culture the Neisseria mucosa of the present invention is not specifically limited, as long as it contains peptone or the like as a nutrient source. Examples thereof can include BHI medium (available from Nippon Becton Dickinson Company, Ltd.).

The culture method is not particularly limited, as long as the conditions are good for the bacterial cells of Neisseria mucosa to grow. Examples thereof include culturing at from 20 to 40°C, preferably from 35 to 38°C, under aerobic conditions, preferably in the presence of 5% CO₂, for from 16 to 72 hours.

In the case of using a bacterial cell culture of Neisseria mucosa in the present invention, Neisseria mucosa is preferably cultured at 37°C in the presence of 5% CO₂ for 24 hours or more in a culture supernatant of periodontal bacteria such as Porphyromonas gingivalis, for example, in a culture supernatant obtained by anaerobically culturing Porphyromonas gingivalis, centrifuging the culture solution obtained, filter-sterilizing the supernatant obtained, and then adding the supernatant therein, for producing EPS.

As shown in Examples below, the bacterial cells or the bacterial cell culture of Neisseria mucosa has an effect of suppressing infection of gingival epithelial cells with periodontal bacteria typified by Porphyromonas gingivalis while suppressing alveolar bone resorption.

Accordingly, the bacterial cells or the bacterial cell culture of Neisseria mucosa can serve as a periodontal bacterial infection-suppressing agent, an alveolar bone resorption-suppressing agent, or an agent for preventing or ameliorating periodontal diseases, and the bacterial cells or the bacterial cell culture of Neisseria mucosa can be used for producing a periodontal bacterial infection-suppressing agent, an alveolar bone resorption-suppressing agent, or an agent for preventing or ameliorating periodontal diseases.

Further, since the bacterial cells or the bacterial cell culture of Neisseria mucosa suppresses infection with periodontal bacteria and further suppresses alveolar bone resorption, they can be used for preventing or ameliorating periodontal diseases. Here, such use can be administration to human or non-human animals or use in specimens derived from them, or may be therapeutic use or non-therapeutic use. Here, examples of non-human animals include non-human mammals, amphibians, and cartilaginous fishes. Examples of the non-human mammals include apes, other primates, mice, rats, horses, cows, pigs, sheep, dogs, cats, hamsters, and companion animals.

In the present invention, the "periodontal diseases" mean inflammatory diseases in which gingival inflammation is caused by bacteria in dental plaque, and encompass gingivitis and periodontitis. The gingivitis is a condition in which periodontal disease bacteria in dental plaque infect the periodontal tissue, to cause gingival inflammation. This gingivitis condition left untreated, resulting in exacerbation of inflammation, may not only cause gingival swelling and hemorrhage, but also allow the periodontal pockets to be deeper, thereby shifting to the periodontitis condition. The progression of periodontitis causes gingival retraction and resorption of the alveolar bone which supports the roots, eventually leading to loss of the teeth.

Further, "suppressing periodontal bacterial infection" means to suppress infection of the gingival cells with periodontal bacteria. Examples of the periodontal bacteria include Fusobacterium nucleatum, Prevotella intermedia, Treponema denticola, Tannerella forsythia, Aggregatibacter actinomycetemcomitans, Campylobacter rectus, and Selenomonas sputigena, in addition to Porphyromonas gingivalis.

Further, "suppressing alveolar bone resorption" means to suppress destruction (resorption) of the alveolar bone, which is the bone supporting the teeth.

In this description, "preventing" means to prevent or delay the onset of a disease or a symptom in an individual, or to reduce the risk of the onset of a disease or a symptom in an individual.

Further, "ameliorating" means to improve a disease, a symptom, or a condition, to prevent or delay deterioration of a disease, a symptom, or a condition, or to reverse, prevent, or delay progression of a disease or a symptom, and includes a concept of so-called "treatment".

The periodontal bacterial infection-suppressing agent, the alveolar bone resorption-suppressing agent, or the agent for preventing or ameliorating periodontal diseases of the present invention itself can be a pharmaceutical product, a quasi-drug, a food, or an oral composition for suppressing infection with periodontal bacteria, suppressing resorption of the alveolar bone, for preventing or ameliorating periodontal diseases, and thus can serve as a material or a formulation to be used by being mixed in the pharmaceutical product, the quasi-drug, the food, or the oral composition.

The food includes foods with a concept to prevent or ameliorate periodontal diseases and halitosis, foods labeled with the concept, as required, foods with function claims, foods for specified health uses, foods for patients, and supplements.

The form used as a pharmaceutical product is preferably the form of oral administration. Examples of the dosage form include various forms including liquid formulations; solid formulations such as tablets, granules, fine granules, powder formulations, and tablets; or capsules, oral sprays, and troches encapsulating the liquid or solid formulations. The pharmaceutical formulations in such various dosage forms can be prepared appropriately in combination with other pharmaceutically acceptable excipients, binders, extenders, disintegrants, surfactants, lubricants, dispersants, buffers, preservatives, flavors, fragrances, coating agents, carriers, and diluents, as long as the actions of the bacterial cells or the bacterial cell culture of Neisseria mucosa are not prevented.

Examples of the form when used as a food include supplements in the same forms as the aforementioned orally administered formulations (tablets, capsules, syrups, and the like), in addition to beverages such as fruit juices or vegetable juices, carbonated beverages, tea beverages, milk beverages, fermented milk, fermented fruit juices, fermented vegetable juices, alcoholic beverages, and soft drinks; and various foods such as jelly foods, various snacks, baked confectioneries, cakes, chocolates, jams, bread, gums, candies, soups, pickles, and Tsukudani (preservable food boiled down in soy sauce). Such foods can be prepared according to a conventional method by appropriately combining the bacterial cells or the bacterial cell culture of Neisseria mucosa with any food material or another active component, or an acceptable additive for foods (for example, a solvent, a softener, an oil, an emulsifier, a preservative, an acidulant, a sweetener, a bittering agent, a pH adjuster, a stabilizer, a colorant, an ultraviolet absorber, an antioxidant, a humectant, a thickener, a fixing agent, a dispersant, a fluidity improver, a moisturizing agent, a fragrance, a seasoning, and a flavor modifier), or the like.

Specific examples of the form when used as an oral composition include dental rinses, mouthwashes, toothpaste, toothpowders, mouthwashes, oral ointments, gel agents, tablets, granules, fine granules, gummy jellies, troches, tablets, capsules, candies, and chewing gums, preferably, toothpaste, dental rinses, gummy jellies, and troches. The oral composition can be prepared according to a conventional method, for example, by appropriately mixing a binding agent, a humectant, an abrasive, a sweetener, a preservative, a surfactant, a fragrance, or the like.

The content of the bacterial cells of Neisseria mucosa in the above-mentioned pharmaceutical product, the food, or the oral composition is not specifically limited and may be appropriately adjusted depending on the daily dose or the like. Examples thereof include 5 × 10⁸ or more, preferably 1 × 10¹⁰ or more, and 1 × 10¹³ or less, more preferably 1 × 10¹¹ or more and 1 × 10¹² or less, on the basis of the bacterial cells of Neisseria mucosa. The content of the bacterial cell culture of Neisseria mucosa is not specifically limited but may be appropriately adjusted depending on the daily dose or the like. Examples thereof include 0.1 mg or more, preferably 100 mg or more, more preferably 500 mg or more, and 5,000 mg or less, preferably 2,500 mg or less, more preferably 1,000 mg or less.

In the above-mentioned pharmaceutical product, the food, or the oral composition, the dose of the bacterial cells of Neisseria mucosa can be appropriately determined depending on various conditions such as the body weight, age, gender, or symptom of the patient. Examples thereof can include 1 × 10⁸ or more, preferably 1 × 10¹⁰ or more, and 1 × 10¹³ or less, more preferably 1 × 10¹¹ or more and 1 × 10¹² or less, as a daily dose, on the basis of the number of the bacteria of Neisseria mucosa. Further, examples of the dose of the bacterial cell culture of Neisseria mucosa include 0.1 mg or more, preferably 100 mg or more, more preferably 500 mg or more, and 5,000 mg or less, preferably 2,500 mg or less, more preferably 1,000 mg or less, as a dry weight of one adult per day when freeze-dried.

Examples of the subject to receive administration of, or ingest, the periodontal bacterial infection-suppressing agent, the periodontal bacterial infection-suppressing food, the alveolar bone resorption-suppressing agent, the alveolar bone resorption-suppressing food, the agent for preventing or ameliorating periodontal diseases, or the food for preventing or ameliorating periodontal diseases of the present invention preferably include humans infected with periodontal bacteria, humans with the gingival inflammation such as gingivitis and periodontitis, further humans with alveolar bone resorption, humans wishing to prevent the onset of the gingival inflammation and alveolar bone resorption, for example, humans with or at risk of gingival swelling and hemorrhage, humans with or at risk of gingival retraction and alveolar bone resorption, and the like.

Regarding the aforementioned embodiments, the following aspects are further disclosed in the present invention.
<1> A periodontal bacterial infection-suppressing agent comprising bacterial cells or a bacterial cell culture of Neisseria mucosa as an active component.
<2> An alveolar bone resorption-suppressing agent comprising bacterial cells or a bacterial cell culture of Neisseria mucosa as an active component.
<3> An agent for preventing or ameliorating periodontal diseases comprising bacterial cells or a bacterial cell culture of Neisseria mucosa as an active component.
<4> A periodontal bacterial infection-suppressing food comprising bacterial cells or a bacterial cell culture of Neisseria mucosa as an active component.
<5> An alveolar bone resorption-suppressing food comprising bacterial cells or a bacterial cell culture of Neisseria mucosa as an active component.
<6> A food for preventing or ameliorating periodontal diseases comprising bacterial cells or a bacterial cell culture of Neisseria mucosa as an active component.
<7> Use of bacterial cells or a bacterial cell culture of Neisseria mucosa for producing a periodontal bacterial infection-suppressing agent.
<8> Use of bacterial cells or a bacterial cell culture of Neisseria mucosa for producing an alveolar bone resorption-suppressing agent.
<9> Use of bacterial cells or a bacterial cell culture of Neisseria mucosa for producing an agent for preventing or ameliorating periodontal diseases.
<10> Use of bacterial cells or a bacterial cell culture of Neisseria mucosa for producing a periodontal bacterial infection-suppressing food.
<11> Use of bacterial cells or a bacterial cell culture of Neisseria mucosa for producing an alveolar bone resorption-suppressing food.
<12> Use of bacterial cells or a bacterial cell culture of Neisseria mucosa for producing a food for preventing or ameliorating periodontal diseases.
<13> Bacterial cells or a bacterial cell culture of Neisseria mucosa for use in suppressing periodontal bacterial infection.
<14> Bacterial cells or a bacterial cell culture of Neisseria mucosa for use in suppressing alveolar bone resorption.
<15> Bacterial cells or a bacterial cell culture of Neisseria mucosa for use in preventing or ameliorating periodontal diseases.
<16> Non-therapeutic use of bacterial cells or a bacterial cell culture of Neisseria mucosa for suppressing periodontal bacterial infection.
<17> Non-therapeutic use of bacterial cells or a bacterial cell culture of Neisseria mucosa for suppressing alveolar bone resorption.
<18> Non-therapeutic use of bacterial cells or a bacterial cell culture of Neisseria mucosa for preventing or ameliorating periodontal diseases.
<19> A method for suppressing periodontal bacterial infection, comprising administering or bacterial cells or a bacterial cell culture of Neisseria mucosa to a subject in need thereof, or allowing the subject to ingest bacterial cells or a bacterial cell culture of Neisseria mucosa, in an effective amount.
<20> A method for suppressing alveolar bone resorption, comprising administering or bacterial cells or a bacterial cell culture of Neisseria mucosa to a subject in need thereof, or allowing the subject to ingest bacterial cells or a bacterial cell culture of Neisseria mucosa, in an effective amount.
<21> A method for preventing or ameliorating periodontal diseases, comprising administering bacterial cells or a bacterial cell culture of Neisseria mucosa to a subject in need thereof, or allowing the subject to ingest bacterial cells or a bacterial cell culture of Neisseria mucosa, in an effective amount.
<22> In <1>, <4>, <7>, <10>, <13>, <16>, or <19>, periodontal bacteria are preferably one or more selected from the group consisting of Porphyromonas gingivalis, Fusobacterium nucleatum, Prevotella intermedia, Treponema denticola, Tannerella forsythia, Aggregatibacter actinomycetemcomitans, Campylobacter rectus, and Selenomonas sputigena.
<23> In from <1> to <22>, the bacterial cell culture is preferably a culture obtained by adding a culture supernatant of Porphyromonas gingivalis to a culture solution of Neisseria mucosa, followed by culturing, or a culture containing EPS which is obtained by adding a culture supernatant of Porphyromonas gingivalis to a culture solution of Neisseria mucosa, followed by culturing, and removing bacterial cells and medium components.
<24> A method for producing a culture containing EPS of Neisseria mucosa, comprising: adding a culture supernatant of Porphyromonas gingivalis to a culture solution of Neisseria mucosa, followed by culturing; and then removing bacterial cells and medium components, followed by collecting.

### Examples

Example 1: Effect of suppressing infection with periodontal bacteria (Porphyromonas gingivalis, Fusobacterium nucleatum, Treponema denticola, and Prevotella intermedia) by Neisseria mucosa

### (1) Cell culture conditions

Human gingival epithelial cells (Ca9-22) were obtained from the JCRB cell bank (JCRB0625). Using DMEM (Gibco, containing 10% v/v FBS, 1% v/v Penicillin-Streptomycin), Ca9-22 was cultured at a temperature of 37°C with a CO₂ concentration of 5%.

### (2) Bacteria culture conditions

Porphyromonas gingivalis and Fusobacterium nucleatum were obtained from the ATCC, and Treponema denticola and Prevotella intermedia were obtained from the Japan Collection of Microorganisms (JCM) (Table 1). Using Anaero Columbia agar (Nippon Becton Dickinson Company, Ltd. (which will be hereinafter referred to as Japan BD)), colonies were cultured under anaerobic conditions at a temperature of 37°C using AnaeroPack/Kenki (MITSUBISHI GAS CHEMICAL COMPANY, INC.) using GAM bouillon medium (available from Nissui Pharmaceutical Co., Ltd., containing 5.0 µg/mL Hemin, 17.4 µg/mL K₂HPO₄, 1.0 µg/mL Vitamin K) as a liquid culture.

Six strains of the genus Neisseria were obtained from the ATCC or the JCM (Table 1). Colonies were cultured using Brain Heart Infusion (BHI) Agar medium (Japan BD) and BHI medium (Japan BD) as liquid cultures at a temperature of 37°C.

Bacterial cells pre-cultured for 24 hours and then liquid-cultured at 100-fold dilution for another 24 hours were used as Porphyromonas gingivalis, and bacterial cells pre-cultured for 24 hours and then liquid-cultured at 1,000-fold dilution for another 24 hours were used as Fusobacterium nucleatum, Treponema denticola and Prevotella intermedia. Bacterial cells pre-cultured for 24 hours and then liquid-cultured at 100-fold dilution for another 48 hours were used as the bacteria of the genus Neisseria.

**[Table 1]**

| | Bacterial type | Strain |
|---|---|---|
| Bacteria of the genus Neisseria | Neisseria mucosa | JCM 12992 (ATCC 19696) |
| | Neisseria sicca | ATCC 29256 |
| | Neisseria flava | ATCC 14221 |
| | Neisseria subflava | ATCC 49275 |
| | Neisseria flavescens | ATCC 13120 |
| | Neisseria elongata | ATCC 25295 |
| Periodontal bacteria | Fusobacterium nucleatum | ATCC 23726 |
| | Prevotella intermedia | JCM 11150 (ATCC25611) |
| | Treponema denticola | JCM8153 (ATCC35405) |
| | Porphyromonas gingivalis | ATCC 33277 |

### (3) Reagent

Carboxyfluorescein diacetate succinimidyl ester (CFSE) as a labeled reagent was purchased from DOJINDO LABORATORIES and dissolved in DMSO, and then used at a final concentration of 10 µM.

### (4) Fluorescence labeling method of bacterial cells

The culture solutions of Porphyromonas gingivalis, Fusobacterium nucleatum, Treponema denticola, and Prevotella intermedia were subjected to centrifugation at 4°C and 3,500 g for 10 minutes to collect respective bacterial cells precipitated. The bacterial cells collected were washed using PBS(-) and then DMEM (Gibco) by centrifugation at 4°C and 3,500 g for 5 minutes, then adjusted to OD600 = 1.0, and incubated in an 10 µM CFSE solution (dissolving DMEM) at 37°C in the dark for 30 minutes, for fluorescence labeling. The fluorescently labeled bacterial cells were washed twice using DMEM (Gibco) by centrifugation at 4°C and 3,500 g for 5 minutes, and then the turbidity of the suspension of the bacterial cells was adjusted, to produce Porphyromonas gingivalis, Fusobacterium nucleatum, Treponema denticola, and Prevotella intermedia labeled with CFSE.

### (5) Infection method

After the medium was removed from the culture plate of Ca9-22 (seeded at 0.3 × 10⁶ cells/well in a 12-well plate) cultured for 24 hours, followed by washing with PBS(-), various bacteria of the genus Neisseria or Neisseria mucosa suspended in DMEM (Gibco) were added at MOI = from 20 to 500, and Porphyromonas gingivalis or Fusobacterium nucleatum, Treponema denticola, and Prevotella intermedia labeled with CFSE were added at MOI = 500 and cultured for 2 hours, for infection.

### (6) Analysis method of infected cells

The medium was removed from each Ca9-22 infected with Porphyromonas gingivalis or Fusobacterium nucleatum, Treponema denticola, and Prevotella intermedia labeled with CFSE, followed by washing twice with PBS(-), and infected cells were dissociated using 0.25% Trypsin-EDTA(Gibco), to collect the cells into a 1.5-mL tube. To the cells collected, was added 400 µL of a 4% paraformaldehyde phosphate buffer solution (Wako), followed by pipetting and a fixation treatment at 4°C for 30 minutes. 800 µL of PBS (containing 2% FBS) was added thereto, followed by pipetting and centrifugation at 4°C and 600 g for 3 minutes, and suspended in 400 µL of PBS (containing 2% FBS), to give an analysis sample.

For the analysis, Flow cytometry (BD, FACSVerse) was used. The mean fluorescence intensity (MFI) was calculated from the fluorescence intensities obtained through the FITC channels of 500,000 cells gated based on forward scatter (FSC) and side scatter (SSC), and the cell infectivity was evaluated by detecting cells to which the Porphyromonas gingivalis or Fusobacterium nucleatum, Treponema denticola, and Prevotella intermedia fluorescently labeled was attached or infected therewith.

### (7) Statistical analysis

The results obtained were each expressed as a mean value ± the standard error (Mean ± standard error (SE)). For the test of the difference between mean values, Dunnett's test or Tukey-Kramer test was performed using a statistics analysis software SPSS (IBM), and the statistical difference was determined to be significant when the P value was less than 0.05 (P < 0.05).

### (8) Results

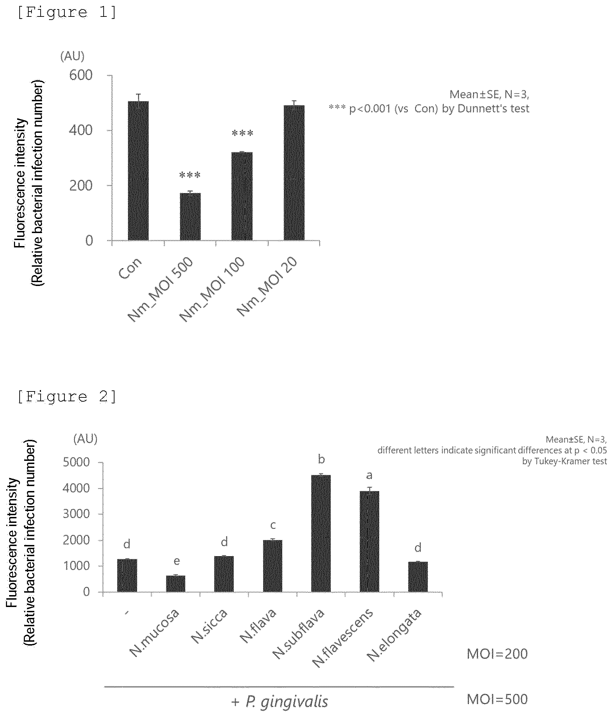

1) Figure 1 shows the effect of suppressing infection with Porphyromonas gingivalis by Neisseria mucosa.

From Figure 1, it was confirmed that Neisseria mucosa inhibits infection with Porphyromonas gingivalis to gingival epithelial cells depending on the number of bacterial cells.

2) Figure 2 shows the effect of suppressing infection with Porphyromonas gingivalis by the bacteria of the genus Neisseria.

From Figure 2, it was confirmed that Neisseria mucosa among the bacteria of the genus Neisseria specifically inhibits the infection with Porphyromonas gingivalis.

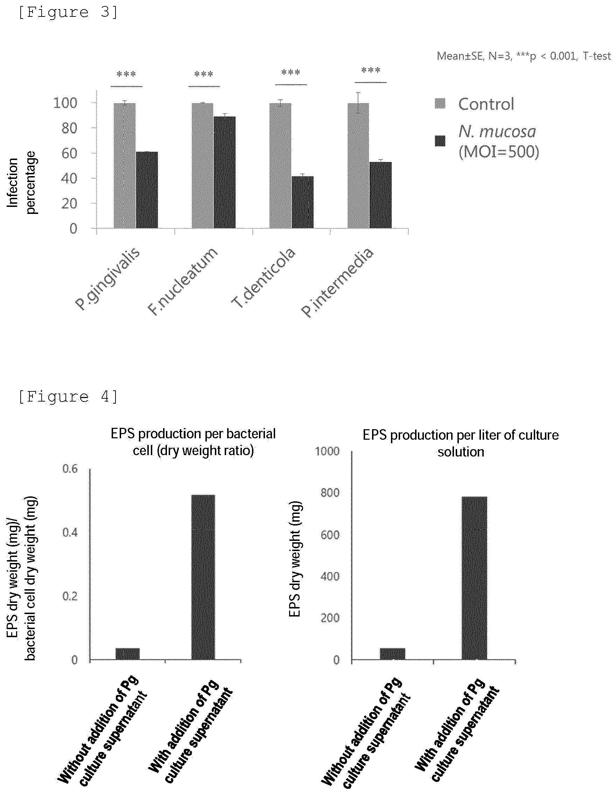

3) Figure 3 shows the effect of suppressing infection with Fusobacterium nucleatum, Treponema denticola, and Prevotella intermedia by Neisseria mucosa.

From Figure 3, it was confirmed that Neisseria mucosa also inhibits infection with periodontal bacteria (Fusobacterium nucleatum, Treponema denticola, and Prevotella intermedia) other than Porphyromonas gingivalis.

Example 2: Effect of suppressing infection with periodontal bacteria (Porphyromonas gingivalis, Fusobacterium nucleatum, Treponema denticola, and Prevotella intermedia) by bacterial cell culture of Neisseria mucosa

### (1) Preparation of bacterial cell culture of Neisseria mucosa

Neisseria mucosa was cultured at a temperature of 37°C for 48 hours using 300 mL of BHI medium, to prepare the culture solution of Neisseria mucosa.

In the group without addition of the culture supernatant of Porphyromonas gingivalis, GAM bouillon medium was added to the culture solution of Neisseria mucosa to a final concentration of 5%, followed by culturing at a temperature of 37°C for 48 hours, to collect 20 mL of the culture solution of Neisseria mucosa into a 50-mL tube. In the group with addition of the culture supernatant of Porphyromonas gingivalis, the culture supernatant of Porphyromonas gingivalis prepared to a final concentration of 5% was added to the culture solution of Neisseria mucosa, followed by culturing at a temperature of 37°C for 48 hours, to collect 20 mL of the culture solution of Neisseria mucosa into a 50-mL tube. The culture supernatant of Porphyromonas gingivalis was prepared by adding a preculture solution of Porphyromonas gingivalis to GAM bouillon medium (available from Nissui Pharmaceutical Co., Ltd.) to a final concentration of 1%, culturing the mixture at 37°C for 24 hours under anaerobic conditions, centrifuging the culture solution obtained at 13,000 g and 4°C for 15 minutes to remove bacterial cells, and filter-sterilizing the supernatant fraction with a 0.22-µm filter.

In order to remove the medium components from the culture solution of Neisseria mucosa, the supernatant obtained by centrifugation at 16,000 g and 4°C for 30 minutes was discarded, and 20 mL of ultrapure water was added thereto, followed by washing. The washing was repeated 6 times. After washing, centrifugation was performed in the same manner, and the bacterial cell culture containing bacterial cells and EPS precipitated was dispersed in ultrapure water, followed by ultrasonic crushing.

After crushing in the ultrasonic crushing conditions: with BRANSON SONIFIER 150 (power 4 and 5 sets of crushing for 20 seconds and then cooling for 20 seconds), bacterial cells were precipitated by centrifugation at 16,000 g and 4°C for 30 minutes, to collect the supernatant into a new 50-mL tube.

In order to completely remove the bacterial cells, the supernatant collected was again centrifuged at 16,000 g and 4°C for 30 minutes, to collect the supernatant.

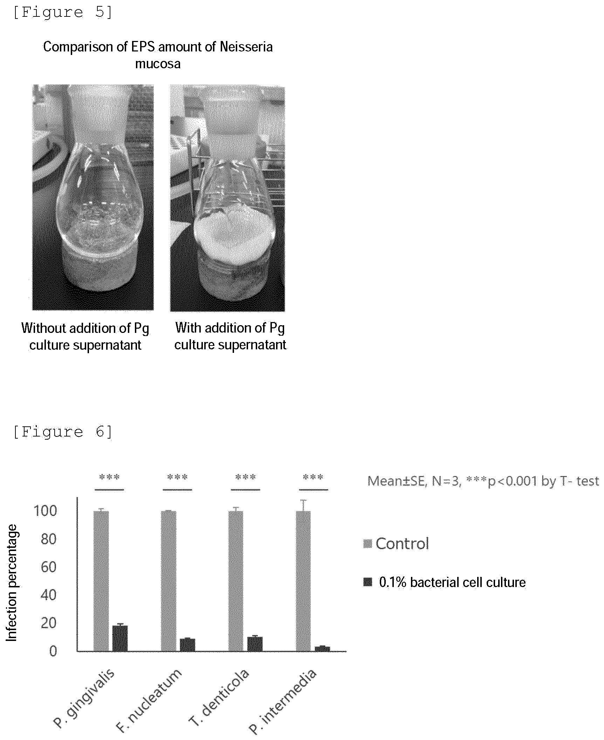

The supernatant collected was freeze-dried, to obtain a bacterial cell culture of Neisseria mucosa from which the bacterial cells and the medium components were removed (the group with addition of culture supernatant of Porphyromonas gingivalis and the group without addition of culture supernatant of Porphyromonas gingivalis). Figure 4 and Figure 5 show the results in which the yield of the bacterial cell culture was increased by addition of the culture supernatant of Porphyromonas gingivalis.

### (2) The bacterial cell culture of Neisseria mucosa prepared in (1) was dissolved in DMEM (Gibco) to a final concentration of 0.1%, and the effect of suppressing infection of the gingival epithelial cells with Porphyromonas gingivalis, Fusobacterium nucleatum, denticola Prevotella intermedia, and Treponema denticola was checked by the same method as in Example 1. Figure 6 shows the results.

### (3) Results

From Figure 6, it was confirmed that the bacterial cell culture of Neisseria mucosa had the effect of suppressing infection with periodontal bacteria including Porphyromonas gingivalis, Fusobacterium nucleatum, Treponema denticola, and Prevotella intermedia to the gingival epithelial cells.

Example 3: Effect of suppressing alveolar bone resorption by Neisseria mucosa using mouse periodontal disease model mice

### (1) Subject animals

As subject animals, 4 week-old female BALB/c mice (Japan SLC) were used. The mice were bred under conditions of the light-dark cycle (light period; from AM7 to PM7) at room temperature 23 ± 2°C and 55 ± 10% for 12 hours. During the one-week preliminary breeding period, the mice were allowed to freely drink tap water and freely eat the animal sample CE-2 solid diet (CLEA Japan, Inc.).

### (2) Bacteria culture conditions

Using the same strains of Porphyromonas gingivalis and, Neisseria mucosa, and Neisseria flavescens as in Example 1, bacteria were cultured by the same method as in Example 1.

### (3) Porphyromonas gingivalis-infected mouse periodontitis model

With reference to the method of Kobayashi, et al. (R. Kobayashi, et al., J Dent Res (2011) 90 (5): 653-658), a Porphyromonas gingivalis-infected periodontitis model was used to evaluate. The mice were preliminary bred for one week and then were bred for 10 days while being allowed to drink tap water containing 2 mg/mL Sulfamethoxazole (FUJIFILM Wako Pure Chemical Corporation) and 0.4 mg/mL Trimethoprim (Toronto Research Chemicals). Thereafter, the mice were allowed to freely drink the tap water for 4 days and then assigned to (1) a non-infection group, (2) a Porphyromonas gingivalis single infection group, (3) a Porphyromonas gingivalis and Neisseria mucosa co-infection group, and (4) a Porphyromonas gingivalis and Neisseria flavescens co-infection group, so that the average body weight was uniform between the groups. After the assignment, suspensions in 100 µL of 2% sodium carboxymethyl cellulose (SIGMA)/PBS (FUJIFILM Wako Pure Chemical Corporation), which were prepared so that Porphyromonas gingivalis was 10⁹ CFU, and Neisseria mucosa and Neisseria flavescens were each 10⁸ CFU, were added dropwise into the oral cavity of the mice under isoflurane inhalation anesthesia once a day, 15 times in total (5 times a week for a total of 3 weeks), to infect the mice, thereby inducing periodontitis. Similarly, 100 µL of 2% CMC/PBS was added dropwise daily to the Sham group that did not induce periodontitis. 27 days after the day of the final infection, the mice were euthanized by exsanguination under isoflurane inhalation anesthesia, and then the maxillas of the mice were collected.

### (4) Measurement method of alveolar bone resorption

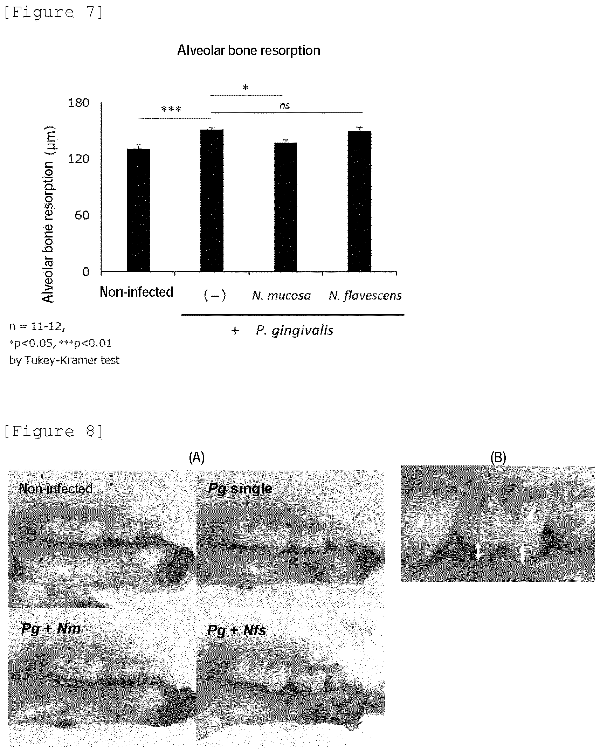

The alveolar bone resorption was measured with reference to the method of Jeong, et al. (Jeong SH, et al. Exp Mol Med (2018) 23 ;50 (3): e460). Excess tissue was removed from each mouse maxilla and transferred to a 24-well plate. Thereafter, the tissue was defleshed by heat treatment in a microwave oven. 1 mL of 3% hydrogen peroxide (FUJIFILM Wako Pure Chemical Corporation) was added into the 24-well plate containing the jawbone tissue after heating for immersion overnight under room temperature conditions. After washing with pure water, the jawbone tissue was stained with a 1% Methylene Blue solution (FUJIFILM Wako Pure Chemical Corporation) for 1 minute and then sufficiently washed with pure water. The sample after staining was captured by a stereomicroscope, and the distance from the Cementoenamel junction (CEJ) of the maxillary second premolar to the alveolar bone crest (ABC) was measured at two points (arrow portions in Figure 8(B)) with Image J, to evaluate the alveolar bone resorption by calculating the average length of the samples.

### (5) Statistical analysis

The results obtained were each expressed as a mean value ± the standard error (Mean ± standard error (SE)). For the test of the difference between mean values, Tukey-Kramer test was performed using a statistics analysis software SPSS (IBM), and the statistical difference was determined to be significant when the P value was less than 0.05 (P < 0.05).

### (6) Results

Figure 7 shows the effect of suppressing alveolar bone resorption by Neisseria mucosa, and Figure 8(A) includes the photographs of the mouse maxillas after the test.

From Figure 7, it was confirmed that the alveolar bone resorption induced by the infection with Porphyromonas gingivalis is suppressed by the co-infection of Neisseria mucosa. Meanwhile, no effect of suppressing alveolar bone resorption was confirmed in co-infection with Neisseria flavescens, which belongs to the same genus Neisseria.

## Claims

1. A periodontal bacterial infection-suppressing agent, comprising bacterial cells or a bacterial cell culture of Neisseria mucosa as an active component.

2. An alveolar bone resorption-suppressing agent, comprising bacterial cells or a bacterial cell culture of Neisseria mucosa as an active component.

3. An agent for preventing or ameliorating periodontal diseases, comprising bacterial cells or a bacterial cell culture of Neisseria mucosa as an active component.

4. A periodontal bacterial infection-suppressing food, comprising bacterial cells or a bacterial cell culture of Neisseria mucosa as an active component.

5. An alveolar bone resorption-suppressing food, comprising bacterial cells or a bacterial cell culture of Neisseria mucosa as an active component.

6. A food for preventing or ameliorating periodontal diseases, comprising bacterial cells or a bacterial cell culture of Neisseria mucosa as an active component.

7. The agent of claim 1 or the food of claim 4, wherein periodontal bacteria are one or more selected from the group consisting of Porphyromonas gingivalis, Fusobacterium nucleatum, Prevotella intermedia, Treponema denticola, Tannerella forsythia, Aggregatibacter actinomycetemcomitans, Campylobacter rectus, and Selenomonas sputigena.

8. The agent of claim 1, 2, 3, or 7, or the food of claim 4, 5, 6, or 7, wherein the bacterial cell culture is a culture obtained by adding a culture supernatant of Porphyromonas gingivalis to a culture solution of Neisseria mucosa, followed by culturing, or a culture containing EPS which is obtained by adding a culture supernatant of Porphyromonas gingivalis to a culture solution of Neisseria mucosa, followed by culturing, and removing bacterial cells and medium components.

9. A method for producing a culture containing EPS of Neisseria mucosa, comprising:
adding a culture supernatant of Porphyromonas gingivalis to a culture solution of Neisseria mucosa, followed by culturing; and then
removing bacterial cells and medium components, followed by collecting.

10. Use of bacterial cells or a bacterial cell culture of Neisseria mucosa for producing a periodontal bacterial infection-suppressing agent.

11. Use of bacterial cells or a bacterial cell culture of Neisseria mucosa for producing an alveolar bone resorption-suppressing agent.

12. Use of bacterial cells or a bacterial cell culture of Neisseria mucosa for producing an agent for preventing or ameliorating periodontal diseases.

13. Use of bacterial cells or a bacterial cell culture of Neisseria mucosa for producing a periodontal bacterial infection-suppressing food.

14. Use of bacterial cells or a bacterial cell culture of Neisseria mucosa for producing an alveolar bone resorption-suppressing food.

15. Use of bacterial cells or a bacterial cell culture of Neisseria mucosa for producing a food for preventing or ameliorating periodontal diseases.

16. Bacterial cells or a bacterial cell culture of Neisseria mucosa for use in suppressing periodontal bacterial infection.

17. Bacterial cells or a bacterial cell culture of Neisseria mucosa for use in suppressing alveolar bone resorption.

18. Bacterial cells or a bacterial cell culture of Neisseria mucosa for use in preventing or ameliorating periodontal diseases.

19. Non-therapeutic use of bacterial cells or a bacterial cell culture of Neisseria mucosa for suppressing periodontal bacterial infection.

20. Non-therapeutic use of bacterial cells or a bacterial cell culture of Neisseria mucosa for suppressing alveolar bone resorption.

21. Non-therapeutic use of bacterial cells or a bacterial cell culture of Neisseria mucosa for preventing or ameliorating periodontal diseases.

22. A method for suppressing periodontal bacterial infection, comprising administering bacterial cells or a bacterial cell culture of Neisseria mucosa to a subject in need thereof, or allowing the subject to ingest bacterial cells or a bacterial cell culture of Neisseria mucosa, in an effective amount.

23. A method for suppressing alveolar bone resorption, comprising administering bacterial cells or a bacterial cell culture of Neisseria mucosa to a subject in need thereof, or allowing the subject to ingest bacterial cells or a bacterial cell culture of Neisseria mucosa, in an effective amount.

24. A method for preventing or ameliorating periodontal diseases, comprising administering bacterial cells or a bacterial cell culture of Neisseria mucosa to a subject in need thereof, or allowing the subject to ingest bacterial cells or a bacterial cell culture of Neisseria mucosa, in an effective amount.

25. The use of claim 10, 13, or 19, the bacterial cells or bacterial cell culture of claim 16, or the method of claim 22, wherein periodontal bacteria are one or more selected from the group consisting of Porphyromonas gingivalis, Fusobacterium nucleatum, Prevotella intermedia, Treponema denticola, Tannerella forsythia, Aggregatibacter actinomycetemcomitans, Campylobacter rectus, and Selenomonas sputigena.

26. The use of any one of claims 10 to 15 and 19 to 21, the bacterial cells or bacterial cell culture of any one of claims 16 to 18, or the method of any one of claims 22 to 24, wherein the bacterial cell culture is a culture obtained by adding a culture supernatant of Porphyromonas gingivalis to a culture solution of Neisseria mucosa, followed by culturing, or a culture containing EPS which is obtained by adding a culture supernatant of Porphyromonas gingivalis to a culture solution of Neisseria mucosa, followed by culturing and removing bacterial cells and medium components.
